# EUROPÄISCHE PATENTSCHRIFT

(11) **EP 3 781 127 B1**
(45) Veröffentlichungstag und Bekanntmachung des Hinweises auf die Patenterteilung: **12.01.2022**
(21) Anmeldenummer: 19725032.7
(22) Anmeldetag: 23.04.2019
(51) Int. Cl.: A61K 9/00, A61K 38/21, A61K 47/10, A61K 47/32, A61P 25/00

(54) **MIKRONADELSYSTEM ZUR APPLIKATION VON INTERFERON**
MICRONEEDLE SYSTEM FOR APPLYING INTERFERON
SYSTÈME DE MICROAIGUILLE POUR L'APPLICATION D'INTERFÉRON

(30) Priorität: 19.04.2018 DE 102018109460; 21.06.2018 DE 102018114930
(43) Veröffentlichungstag der Anmeldung: 24.02.2021
(73) Patentinhaber: LTS LOHMANN Therapie-Systeme AG, 56626 Andernach (DE)
(72) Erfinder: HENKE, Stefan, 57548 Kirchen (DE); HENNING, Andreas, 72469 Meßstetten (DE); PRACHT, Rolf, 56203 Höhr-Grenzhausen (DE); BRODKORB, Danny, 56626 Andernach (DE); SCHERR, Sebastian, 56355 Neuhäusel (DE)
(74) Vertreter: Simandi, Claus
(86) Internationale Anmeldenummer: PCT/EP2019/060396
(87) Internationale Veröffentlichungsnummer: WO 2019/202170

(56) Entgegenhaltungen:
- JIANMIN CHEN ET AL: "Dissolving microneedle-based intradermal delivery of interferon-[alpha]-2b", DRUG DEVELOPMENT AND INDUSTRIAL PHARMACY, Bd. 42, Nr. 6, 2. Juni 2016 (2016-06-02), Seiten 890-896, XP55606523, US ISSN: 0363-9045, DOI: 10.3109/03639045.2015.1096282
- MIN WANG ET AL: "Recent advances in the design of polymeric microneedles for transdermal drug delivery and biosensing", LAB ON A CHIP, Bd. 17, Nr. 8, 1. Januar 2017 (2017-01-01) , Seiten 1373-1387, XP55554994, ISSN: 1473-0197, DOI: 10.1039/C7LC00016B

## Beschreibung

Die vorliegende Erfindung betrifft ein Mikronadelsystem (kurz: MNS) zur intradermalen Applikation von Interferon.

Interferone sind körpereigene Botenstoffe, mittels derer unterschiedliche Zellen des Immunsystems miteinander kommunizieren. Beta-Interferon beta-1a wird gentechnisch hergestellt und unterscheidet sich nur geringfügig vom humanen Beta-Interferon. Der Wirkstoff kommt bei schubförmiger und sekundär progredienter Multipler Sklerose (MS) zum Einsatz. Vermutet wird, dass durch den Einsatz von Beta-Interferon die Aktivität von autoreaktiven T-Zellen (Abwehrzellen, die sich gegen körpereigenes Gewebe richten) gehemmt wird und dadurch die Schädigung der Myelin-Substanz, welche die Nervenfasern schützend umgibt, verzögert wird.

Aktuell in der MS-Therapie eingesetzte Beta-Interferonpräparate (z. B. Avonex^{®}, Rebif^{®}) werden mehrmals wöchentlich intramuskulär oder subkutan injiziert. Auf Grund der Selbstmedikation von MS-Patienten, mit den damit verbundenen Risiken von Infektionen und Nadelstichverletzungen, kann die Patienten-Compliance in der Beta-Interferontherapie mit einem selbstauflösenden Microarray signifikant verbessert werden. Ein weiterer Vorteil der intradermalen Applikation von Beta-Interferon kann laut Expertenmeinung die unmittelbare Wirkstoff-Freisetzung in Nähe der immunkompetenten Ziel-Zellen in den oberen Hautschichten liegen, was wiederrum im Gegensatz zur parenteralen Applikation des Wirkstoffs (z. B. in Form einer S. C. Infektion) zu einer Verringerung der häufig auftretenden unerwünschten Nebenwirkungen (grippeähnliche Symptome, Anstieg bestimmter Leberwerte) führen könnte.

Die Haut besteht aus mehreren Schichten. Die äußerste Hautschicht, das *Stratum Corneum,* weist bekannte Sperreigenschaften auf, um ein Eindringen von Fremdsubstanzen in den Körper und ein Austreten von Eigensubstanzen aus dem Körper zu verhindern. Das Stratum Corneum, das eine komplexe Struktur aus kompaktierten keratotischen Zellresten mit einer Dicke von ungefähr 10-30 Mikrometern ist, bildet hierzu eine wasserdichte Membran zum Schutz des Körpers aus. Die natürliche Undurchlässigkeit des Stratum Corneum verhindert das Verabreichen der meisten pharmazeutischen Wirkstoffe und anderer Substanzen über die Hautbarriere im Rahmen einer transdermalen Applikationsform. Langerhans-Zellen werden durchgehend in der basalen Granulaschicht des Epithels gefunden, die eine wichtige Rolle in der anfänglichen Verteidigung des Immunsystems gegen eindringende Organismen spielen.

Mikronadelsysteme (MNS), die aus einem Mikronadelarray (MNA) und gegebenenfalls weiteren Komponenten bestehen, können mittels einer Druckkraft die Mikronadeln (auch: Hautdurchdringungselemente) des Arrays (MNA) gegen die Applikationsstelle auf die Haut pressen, um das Stratum Corneum zu durchdringen und auf diese Weise einen Fluidkanal herzustellen, so dass ein Interferon transdermal appliziert werden kann. Solche Mikronadelarrays (MNA) in Mikronadelsystemen (MNS) als auch deren Herstellung sind im Stand der Technik beschrieben und werden auch als Mikro(nadel)array-Pflaster beschrieben. Ebenfalls ist im Stand der Technik bekannt, dass Proteine, einschließlich Interferon über MNS appliziert werden können (z.B. WO2007030477A2). WO2007030477A2 sieht den Einsatz von Wirkstoffpartikeln vor, die an die Spitze eines Perforators zentrifugiert werden. Chen et al (Drug Dev Ind Pharm, 2016, 42(6), 890-896) offenbart die intradermale Applikation von Interferon.

Daher ist es Aufgabe der vorliegenden Erfindung eine intradermale Applikation von Interferon mittels eines MNS enthaltend ein MNA auf Basis einer geeigneten Formulierung zu ermöglichen.

Überraschender Weise ist Polyvinylpyrrolidon (kurz: PVP) zur Herstellung einer vollständig lösbaren Formulierung für einen MNA zur intradermalen Applikation von Interferon besonders geeignet.

Die erfindungsgemäße Formulierung erlaubt eine hinreichende Festigkeit als auch vollständige Lösbarkeit, so dass für Interferon eine hinreichende Stabilität - auch ohne Stabilisatoren - für die Anwendung und Auflösung des MNA und für die Absorption und Verteilung des Wirkstoffs im Organismus erreicht werden kann.

Die erfindungsgemäße Formulierung bezieht sich neben der Eignung der MNA zur unmittelbaren intradermalen Anwendung von Interferon, ebenfalls auf eine stabile Lagerung der erfindungsgemäßen Formulierung zu einem Interferon-haltigen Mikroarray, insbesondere bei Raumtemperatur für 3 Monate und mehr.

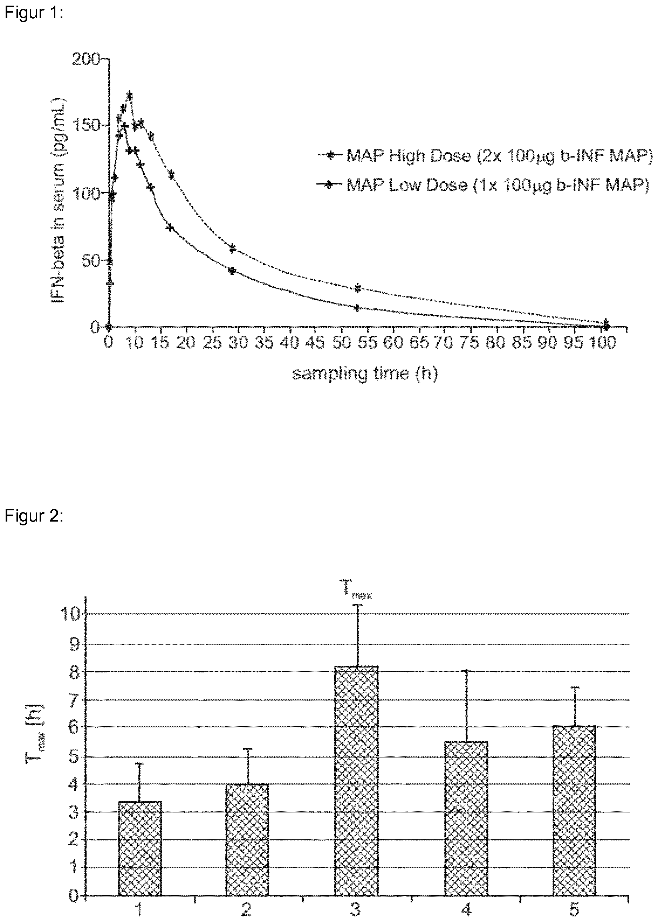

Des Weiteren wurde die erfindungsgemäße Formulierung im Rahmen eines in-vitro Bioassays an einer humanen Zervixkarzinom-Modellzelllinie eingesetzt. Es konnte erfolgreich nachgewiesen werden, dass trotz Zugabe des Enzephalomyokarditis-Virus (EMCV) nicht eine Lyse der humanen Zellen eintritt. Das in den MNA eingebettete Interferon in der erfindungsgemäßen Formulierung wirkt zuverlässig antiviral und weist eine vergleichbare Wirksamkeit wie die Originalpräparate auf. Von daher kann von einem gleichen Therapie-Erfolg ausgegangen werden, wie bei einer Interferon-Injektion (S. C. oder I. M.), siehe Figuren 1-4.

Diese Aufgabe wird daher erfindungsgemäß durch ein Mikronadelarray (MNA) nach Anspruch 1 gelöst, umfassend eine Formulierung aus Polyvinylpyrrolidon zur Verwendung in der intradermalen Applikation von Interferon, wobei Polyvinylpyrrolidon Hauptbestandteil der Formulierung ist.

Gegenstand der Erfindung ist daher ebenfalls ein Mikronadelsystem enthaltend ein MNA zur Verwendung in der intradermalen Applikation von Interferon, wobei Polyvinylpyrrolidon Hauptbestandteil der Formulierung ist.

Daher umfasst die Erfindung ein Mittel aufweisend einen Mikronadelarray, umfassend eine Formulierung aus Polyvinylpyrrolidon zur Verwendung in der intradermalen Applikation von Interferon, wobei Polyvinylpyrrolidon Hauptbestandteil der Formulierung ist.

Ein solches Mittel ist beispielsweise ein Arzneimittel umfassend ein vorstehendes Mikronadelarray (MNA) zur Verwendung in der intradermalen Applikation von Interferon, insbesondere zur Behandlung von Multipler Sklerose (MS) oder zur Interferontherapie.

Besonders bevorzugt ist ein erfindungsgemäßer Mikronadelarray der einen Interferon-Gehalt von 0,1 µg bis 200 µg, insbesondere 10 µg bis 100 µg pro Mikronadelarray aufweist.

Der Begriff "intradermale Applikation (synonym: "intrakutane Applikation") beschreibt erfindungsgemäß die Verabreichung von Interferon aus dem MNA in die Haut und erfordert ein Eindringen der Mikronadeln in die Haut.

Der Begriff "Interferon" umfasst sämtliche bzw. ein oder mehrere Interferone (IFN), alpha-, beta-, gamma-IFN, Tau-Interferon, insbesondere die Interferone beta-1b, Interferon beta-1a zur Behandlung von Multipler Sklerose (MS). Beta-Interferone sind erfindungsgemäß bevorzugt. Interferone sind Proteine oder Glykoproteine, die eine immunstimulierende, vor allem antivirale und antitumorale Wirkung entfalten und körpereigene Zytokine darstellen.

Der Begriff "wobei Polyvinylpyrrolidon Hauptbestandteil der Formulierung ist" bedeutet, dass Polyvinylpyrrolidon neben anderen Hilfsstoffen und dem Wirkstoff Interferon mengenmäßig in Gew. % Hauptbestandteil ist, d.h. die meisten Gew % in einer Zusammensetzung einer vollständig lösbaren Formulierung entfallen auf Polyvinylpyrrolidon.

Ebenfalls offenbart, jedoch nicht beansprucht, ist ein Verfahren zur Durchführung einer intradermalen Applikation von Interferon umfassend
a) Fixierung eines erfindungsgemäßen Mikronadelsystems an der Haut,
b) Penetration eines Mikronadelarrays in die Haut umfassend eine vollständig lösbare Formulierung aus Polyvinylpyrrolidon, wobei Polyvinylpyrrolidon Hauptbestandteil der Formulierung ist.

Im Rahmen dieser Erfindung ist ein Mikronadelsystem ein solches System, welches eine Vorrichtung enthält, die bewirkt, dass der Mikronadelarray zur Verabreichung von Interferon auf der Haut bereitgestellt und intradermal appliziert wird.

Das Mikronadelsystem kann in einer bevorzugten Ausführungsform einen Applikator, wie eine Auslösevorrichtung umfassen, die elektrisch oder mechanisch gesteuert wird. Der Applikator kann beispielsweise einen Kolben aufweisen, der den Mikronadelarray auf die Haut aufsetzt oder aufbringt, so dass die Mikronadeln in die Haut penetrieren.

Die Auslösevorrichtung kann beispielweise eine Pumpe, eine Spritze oder eine Feder umfassen, so dass ein Kolbenstoß mit hinreichender Energie erfolgen kann. Der Kolben kann beliebiger Form und Natur sein und soll in erster Linie bewerkstelligen, dass der Mikronadelarray von einer ersten Stellung in eine zweite Stellung zur Verabreichung des Interferons auf der Haut bereitgestellt wird. Der Applikator kann weiterhin einen Druckknopf oder andere Auslösemechanismen umfassen.

Der Mikronadelarray kann eine Vielzahl von Mikronadeln aufweisen, um Interferon über die Haut oder in die Haut eines Patienten abgeben zu können, wobei der Mikronadelarray auf die Haut des Patienten aufgebracht wird. Jede der Mikronadeln des Mikronadelarrays weist vorzugsweise einen langgestreckten Schaft mit zwei Enden auf, das eine Ende des Schafts ist die Basis der Mikronadel, mit der die Mikronadel an dem flächigen Träger befestigt oder mit der die Mikronadel in den flächigen Träger integriert ist. Das der Basis gegenüberliegende Ende des Schafts ist vorzugsweise spitz zulaufend ausgestaltet, um ein möglichst leichtes Eindringen der Mikronadel in die Haut zu ermöglichen.

Die Mikronadeln können einen Schaft mit einem runden Querschnitt oder einen nicht runden Querschnitt aufweisen, beispielsweise mit einem dreieckigen, viereckigen oder einem polygonalen Querschnitt. Der Schaft kann einen Durchgang oder mehrere Durchgänge aufweisen, die von der Nadelbasis bis zur Nadelspitze oder annähernd zur Nadelspitze verläuft/verlaufen. Die Mikronadeln können als (Wider-)Haken ausgebildet sein, wobei eine oder mehrere dieser Mikronadeln einen oder mehrere solcher Haken aufweisen. Weiterhin können die Mikronadeln schraubenförmig gestaltet und drehbar angeordnet sein und dadurch beim Anwenden einer rotierenden Bewegung das Eindringen in die Haut erleichtern und eine Verankerung in der Haut bewirken (DE 103 53 629 A1), insbesondere in der gewünschten Penetrationstiefe in der Epidermis.

Der Durchmesser einer Mikronadel beträgt üblicherweise zwischen 1 µm und 1000 µm, vorzugsweise zwischen 10 µm und 100 µm. Die Länge einer Mikronadel beträgt üblicherweise zwischen 5 µm und 6.000 µm, insbesondere zwischen 100 µm und 700 µm.

Die Mikronadeln sind mit ihrer Basis an einem flächigen Träger befestigt oder in einen flächigen Träger integriert. Die Mikronadeln sind vorzugsweise im Wesentlichen senkrecht zur Fläche des Trägers stehend angeordnet. Die Mikronadeln können regelmäßig oder unregelmäßig angeordnet sein. Eine Anordnung von mehreren Mikronadeln kann Mikronadeln mit unterschiedlichen Querschnittformen, unterschiedlich dimensionierten Durchmessern und/oder unterschiedlichen Längen aufweisen. Die Anordnung aus mehreren Mikronadeln kann zum Beispiel ausschließlich hohle Mikronadeln aufweisen.

Der Mikronadelarray kann einen flächigen Träger aufweisen, wobei der Träger im Wesentlichen eine scheibenförmige, plattenförmige oder folienförmige Grundform hat. Der Träger kann eine runde, ovale, dreieckige, viereckige oder polygonale Grundfläche haben. Der Träger kann aus unterschiedlichen Materialien hergestellt sein, beispielsweise aus einem Metall, einem keramischen Werkstoff, einem Halbleiter, einem organischen Material, einem Polymer oder einem Komposit.

Gemäß der Ansprüche sind folgende Stoffe in einer Formulierung zur Herstellung der Mikronadeln neben Polyvinylpyrrolidon vorhanden,
solche die aus diesen bestehen oder umfassen: Disaccharid, vorzugsweise Trehalose, nichtionische Tenside, vorzugsweise Polysorbat (ethoxylierte Sorbitanfettsäureester, wie Tween), Polyalkohol, insbesondere Glycerol (Glycerin).

Bevorzugt ist, dass Polyvinylpyrrolidon als Hauptbestandteil mehr als 35 Gew. %, mehr als 45 Gew. %, mehr als 55 Gew. %, mehr als 65 Gew. %, mehr als 75 Gew. %, mehr als 85 Gew. % in der Formulierung beträgt.

Bevorzugt ist, dass das Disaccharid, insbesondere Trehalose, als Nebenbestandteil mehr als 15 Gew. %, mehr als 25 Gew. %, mehr als 35 Gew. % in der Formulierung beträgt.

**Tabelle 1:**

| Stoff I Bezeichnung | Funktion | Konzentration / Dosis in Gew. % |
|---|---|---|
| Beta-Interferon | Wirkstoff | 0,1 µg bis 200 µg pro MNA |
| Polyvinylpyrrolidon (PVP) | Hilfsstoff | 0,1% bis 95% (m/m) |
| Trehalose | Hilfsstoff | 0,1% bis 45 % (m/m) oder bis 95% (m/m) |
| Polysorbat | Hilfsstoff | 0,001% bis 10% (m/m) |
| Glycerol | Hilfsstoff | 0,1% bis 10% (m/m) |
| Acetatpuffer | Losungsmittel | 0,01% bis 10% (m/m) |

Nach Trocknung der flüssigen Formulierung, z.B. zu einem Mikronadelarray (MNA) mit 0,1-20% (m/m) Restwassergehalt, folgt eine entsprechend um den Wasserverlust veränderte Zusammensetzung des MNA.

Nachstehende Beispiele und Figuren dienen zur näheren Erläuterung der Erfindung, ohne jedoch diese einzuschränken.

### Beispiele und Figuren:

Zur Herstellung der erfindungsgemäßen Mikronadeln können die bekannten Verfahren angestrengt werden, wie McCrudden MTC, Alkilani AZ, McCrudden CM, McAlister E, McCarthy HO, Woolfson AD, et al. Design and physicochemical characterisation of novel dissolving polymeric microneedle arrays for transdermal delivery of high dose, low molecular weight drugs. J Control Release. 2014; 180: 71-80.

Die oben beschriebene Formulierung wurde im Rahmen einer präklinischen in-vivo Studie (Tierversuchsmodel: Göttinger Minischwein) eingesetzt.

Figur 1 zeigt die nach Applikation der Mikroarrays erzielten WirkstoffKonzentrationen im Blutserum der Tiere.

Im Rahmen der Entwicklung eines klinischen Prüfpräparats für die Beta-Interferontherapie wurde ein selbstauflösendes Mikronadel- oder Mikroarray-Pflaster (MNP) hergestellt und am Tiermodell Göttinger Minipig getestet.

Die Tierstudie bestätigt die erfolgreiche Anwendung des Mikroarrays und die Auflösung, Absorption und Verteilung der aktiven Wirkstoffsubstanz im Tierversuchsmodell. Die Eignung der Wirkstoff-Formulierung konnte hiermit bestätigt werden.

Obwohl die Plasmakonzentration von Beta-Interferon intradermal niedriger war als nach der S.C.-Injektion, sind Fachleute in der Multiplen Sklerose-Forschung der Meinung, dass durch eine unmittelbare Wirkstoff-Freisetzung in der Nähe der immunkompetenten Ziel-Zellen in den oberen Hautschichten eine höhere Wirksamkeit erzielt werden könnte. Mehr noch, laut Experten, könnte eine geringere Wirkstoffkonzentration einen vergleichbaren Behandlungserfolg erzielen und die häufig auftretenden unerwünschten Nebenwirkungen deutlich minimiert werden. Üblicherweise wird nach einer Beta-Interferon-Injektion (S. C. (subkutan) oder I. M. (intramuskulär)) der gesamte Blutstrom des Patienten innerhalb sehr kurzer Zeit mit einer hohen Konzentration an Zytokinen überflutet. Dies kann bei einer intradermalen Wirkstoffanwendung vermieden werden, da der Wirkstoff zunächst nur die interstitielle Flüssigkeit und das lymphatische System erreicht und hier die entsprechende Wirksamkeit, die Initiierung von ZNS-wirksamen Immunkaskaden, ausgelöst wird.

Figur 2 zeigt die Zeitpunkte bis zum Erreichen des Spitzenplasmaspiegels von Beta-Interferon für die intradermale Applikation mittels Mikroarray Patch im Vergleich zur S. C. Injektion.

Figur 3 zeigt einen antiviralen Beta-Interferon-Aktivitäts-Assay mit Hep-2C-Zellen und infektiösen EMCV nach Ph. Eur. 5.2.3. Beta-Interferon-Microarray (MA) zeigt trotz absoluter Mengengleichheit zum Standard eine höhere Aktivität. Formulierung des Beta-Interferons in halbfester Darreichungsform eines Microarrays bewahrt in-vitro Aktivität.

Aufgrund des Wegfalls von Stabilisatoren und anderen Adjuvantien in der Produktformulierung sind unerwünschte Nebenwirkungen auszuschließen. Eine ausgezeichnete Stabilität des Wirkstoffs in der halbfesten, amorphen Mikroarray-Struktur wurde ohne die Zugabe von häufig verwendeten Stabilisatoren (z. B. Mannitol, HSA oder Arginin) in der MA-Formulierung gezeigt. Darüber hinaus zeigen Stabilitätsuntersuchungen, dass in Mikroarrays formuliertes Beta-Interferon bei Raumtemperatur gelagert werden kann und eine vergleichbare spezifische Aktivität wie andere kühl gelagerte Beta-Interferonpräparate aufweist. Es ist daher vorteilhaft möglich, Beta-Interferon in MA-Formulierung ohne Kühlkette zu lagern und zu transportieren.

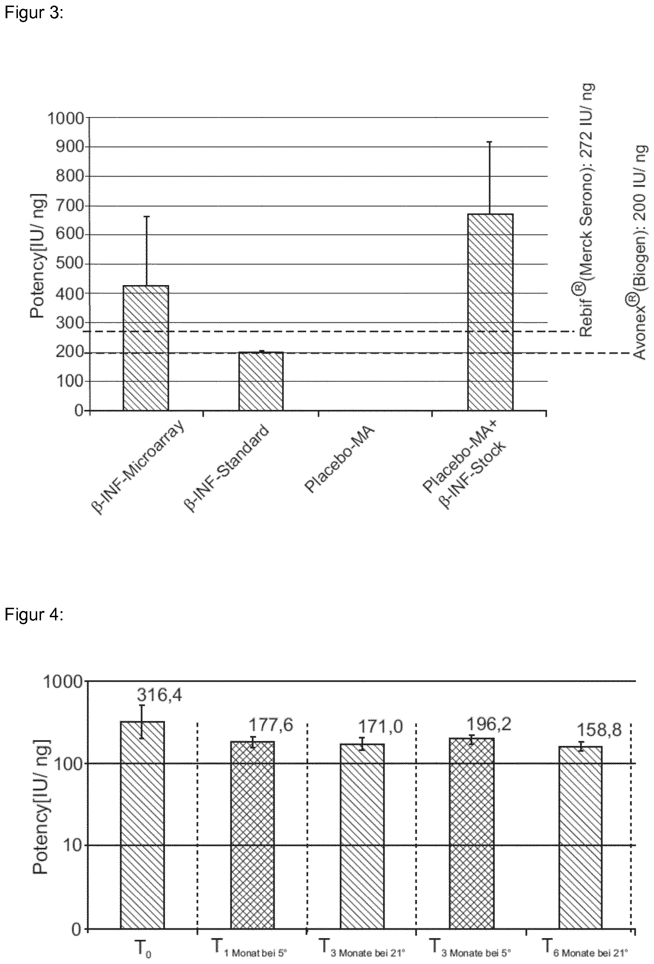

Figur 4 zeigt die Lagerstabilität des Beta-Interferon-Microarrays, und zwar die in-vitro Beta-Interferon-Aktivitätsanalyse nach Herstellung, nach 1 Monat, 3 Monaten und nach 6 Monaten unter Einlagerung bei Raumtemperatur (21°C) und Kühlschranktemperatur (5°C). Die antivirale Wirksamkeit des Beta-Interferons bleibt auch nach 6 Monaten und 21°C-Lagertemperatur im selben Logstufenbereich wie zum Zeitpunkt der Herstellung.

### Legende zu den Figuren:

Figur 1:
   Gemittelte Wirkstoff-Konzentration im Blutserum nach Applikation der Mikroarrays.
Figur 2:
   Zeitpunkte bis zum Erreichen des Spitzenplasmaspiegels von Beta-Interferon für die intradermale Applikation mittels Mikroarray Patch im Vergleich zur S.C. Injektion. Legende: **1** B-IFN-MA 100_g, **2** B-IFN-MA 200_g, **3** Drug substance 40 _g S.C., **4** Avonex 30 _g S.C., **5** Rebif 44 _g S.C.
Figur 3:
   Antiviraler Beta-Interferon-Aktivitäts-Assay mit Hep-2C-Zellen und infektiösen EMCV nach Ph. Eur. 5.2.3 Beta-Interferon-Microarray (B-IFN-MA) zeigt trotz absoluter Mengengleichheit zum Standard eine höhere Aktivität. Formulierung des Beta-Interferons in halbfester Darreichungsform eines Microarrays bewahrt in-vitro Aktivität.
Figur 4:
   Lagerstabilität des Beta-Interferon-Microarrays In-vitro Beta-Interferon-Aktivitätsanalyse nach Herstellung, nach 1 Monat, 3 Monaten und nach 6 Monaten unter Einlagerung bei Raumtemperatur (21°) und Kühlschranktemperatur (5°). Die antivirale Wirksamkeit des 316,4, 177,6, 171,0, 196,2, 158,8 [IU/ng] Beta-Interferons bleibt auch nach 6 Monaten und 21°C-Lagertemperatur im selben Logstufenbereich wie zum Zeitpunkt der Herstellung.

## Patentansprüche

1. Mikronadelarray umfassend eine vollständig lösbare Formulierung aus Polyvinylpyrrolidon zur Verwendung in der intradermalen Applikation von Interferon, **dadurch gekennzeichnet, dass** Polyvinylpyrrolidon Hauptbestandteil der Formulierung ist, und die Formulierung
Disaccharid, nichtionische Tenside, Polyalkohol, insbesondere Glycerin enthält oder
Trehalose und/oder Polysorbat und/oder Glycerin enthält.

2. Mikronadelarray umfassend eine vollständig lösbare Formulierung aus Polyvinylpyrrolidon zur Verwendung nach Anspruch 1, **dadurch gekennzeichnet, dass** Polyvinylpyrrolidon mehr als 45 Gew. % in der Formulierung beträgt.

3. Mikronadelarray umfassend eine vollständig lösbare Formulierung aus Polyvinylpyrrolidon zur Verwendung nach Anspruch 1 oder 2, wobei der Interferon-Gehalt 0,1 µg bis 200 µg pro Mikronadelarray beträgt.

4. Mittel, insbesondere Arzneimittel, aufweisend einen Mikronadelarray umfassend eine vollständig lösbare Formulierung aus Polyvinylpyrrolidon nach einem der Ansprüche 1-3 zur Verwendung in der intradermalen Applikation von Interferon, insbesondere zur Behandlung von Multiple Sklerose oder zur Interferontherapie.

5. Mikronadelarray oder Mittel zur Verwendung in der intradermalen Applikation von Interferon nach einem der vorstehenden Ansprüche, **dadurch gekennzeichnet, dass** eine Formulierung bis 95 Gew. % Polyvinylpyrrolidon und weitere Hilfs- und Zusatzstoffe enthält.

6. Mikronadelarray oder Mittel zur Verwendung in der intradermalen Applikation von Interferon nach einem der vorstehenden Ansprüche, **dadurch gekennzeichnet, dass** eine Formulierung bis 95 Gew.-% Polyvinylpyrrolidon und 0,1 Gew.-% bis 45 Gew.-% Trehalose enthält.

7. Mikronadelarray oder Mittel zur Verwendung in der intradermalen Applikation von Interferon nach einem der vorstehenden Ansprüche, **dadurch gekennzeichnet, dass** eine Formulierung bis 95 Gew.-% Polyvinylpyrrolidon und bis 0,001 Gew.-% bis 10 Gew.-% Polysorbat enthält.

8. Mikronadelarray oder Mittel zur Verwendung in der intradermalen Applikation von Interferon nach einem der vorstehenden Ansprüche, **dadurch gekennzeichnet, dass** eine Formulierung bis 95 Gew.-% Polyvinylpyrrolidon und bis 0,1 Gew.-% bis 10 Gew.-% Glycerin enthält.

9. Mikronadelsystem aufweisend einen Mikronadelarray nach einem der Ansprüche 1 bis 8 und einen Applikator.

10. Mikronadelsystem aufweisend einen Mikronadelarray und einen Applikator nach Anspruch 9, wobei der Applikator eine Auslösevorrichtung umfasst.

## Claims

1. A microneedle array comprising a completely soluble formulation including polyvinylpyrrolidone for use in the intradermal delivery of interferon, **characterized in that** polyvinylpyrrolidone is the major constituent of the formulation, and the formulation comprises
disaccharide, non-ionic surfactants, polyalcohol, in particular glycerin, or trehalose and/or polysorbate and/or glycerin.

2. The microneedle array comprising a completely soluble formulation including polyvinylpyrrolidone for use according to claim 1, **characterized in that** polyvinylpyrrolidone accounts for more than 45 wt. % in the formulation.

3. The microneedle array comprising a completely soluble formulation including polyvinylpyrrolidone for use according to claim 1 or 2, wherein the interferon content is 0.1 µg to 200 µg per microneedle array.

4. A product, in particular a pharmaceutical product, comprising a microneedle array comprising a completely soluble formulation including polyvinylpyrrolidone according to any one of the preceding claims 1 to 3 for use in the intradermal delivery of interferon, and in particular for the treatment of multiple sclerosis or for interferon therapy.

5. A microneedle array or a product for use in the intradermal delivery of interferon according to any one of the preceding claims, **characterized in that** a formulation comprises up to 95 wt.% polyvinylpyrrolidone and further adjuvants and additives.

6. A microneedle array or a product for use in the intradermal delivery of interferon according to any one of the preceding claims, **characterized in that** a formulation comprises up to 95 wt.-% polyvinylpyrrolidone and 0.1 wt.% to 45 wt.% trehalose.

7. A microneedle array or a product for use in the intradermal delivery of interferon according to any one of the preceding claims, **characterized in that** a formulation comprises up to 95 wt.-% polyvinylpyrrolidone and 0.001 wt.% to 10 wt.% polysorbate.

8. A microneedle array or a product for use in the intradermal delivery of interferon according to any one of the preceding claims, **characterized in that** a formulation comprises up to 95 wt.-% polyvinylpyrrolidone and 0.1 wt.% to 10 wt.% glycerin.

9. A microneedle system comprising a microneedle array according to any one of claims 1 to 8, and an applicator.

10. A microneedle system comprising a microneedle array and an applicator according to claim 9, wherein the applicator comprises a trigger device.

## Revendications

1. Matrice de micro-aiguilles comprenant une formulation complètement soluble comprenant de la polyvinylpyrrolidone pour une utilisation dans la délivrance intradermique d'interféron, **caractérisée en ce que** la polyvinylpyrrolidone est le constituant majeur de la formulation, et la formulation comprend un disaccharide, des tensioactifs non ioniques, un polyalcool, en particulier de la glycérine, ou de l'tréhalose et/ou un polysorbate et/ou de la glycérine.

2. Matrice de micro-aiguilles comprenant une formulation complètement soluble incluant de la polyvinylpyrrolidone pour une utilisation selon la revendication 1, **caractérisée en ce que** la polyvinylpyrrolidone représente plus de 45 % en poids dans la formulation.

3. Matrice de micro-aiguilles comprenant une formulation complètement soluble incluant de la polyvinylpyrrolidone pour une utilisation selon la revendication 1 ou 2, dans laquelle la teneur en interféron est de 0,1 µg à 200 µg par matrice de micro-aiguilles.

4. Produit, en particulier un produit pharmaceutique, comprenant un réseau de micro-aiguilles comprenant une formulation complètement soluble incluant de la polyvinylpyrrolidone selon l'une quelconque des revendications précédentes 1 à 3 pour une utilisation dans la délivrance intradermique d'interféron, et en particulier pour le traitement de la sclérose en plaques ou pour la thérapie par interféron.

5. Matrice de micro-aiguilles ou produit destiné à être utilisé pour l'administration intradermique d'interféron selon l'une quelconque des revendications précédentes, **caractérisé en ce qu'**une formulation comprend jusqu'à 95 % en poids de polyvinylpyrrolidone et d'autres adjuvants et additifs.

6. Matrice de micro-aiguilles ou produit destiné à être utilisé pour l'administration intradermique d'interféron selon l'une quelconque des revendications précédentes, **caractérisé en ce qu'**une formulation comprend jusqu'à 95 % en poids de polyvinylpyrrolidone et 0,1 % en poids à 45 % en poids de tréhalose.

7. Matrice de micro-aiguilles ou produit destiné à être utilisé pour l'administration intradermique d'interféron selon l'une quelconque des revendications précédentes, **caractérisé en ce qu'**une formulation comprend jusqu'à 95 % en poids de polyvinylpyrrolidone et 0,001 % en poids à 10 % en poids de polysorbate.

8. Matrice de micro-aiguilles ou produit destiné à être utilisé pour l'administration intradermique d'interféron selon l'une quelconque des revendications précédentes, **caractérisé en ce qu'**une formulation comprend jusqu'à 95 % en poids de polyvinylpyrrolidone et 0,1 % en poids à 10 % en poids de glycérine.

9. Système de micro-aiguilles comprenant un réseau de micro-aiguilles selon l'une quelconque des revendications 1 à 8, et un applicateur.

10. Système de micro-aiguilles comprenant un réseau de micro-aiguilles et un applicateur selon la revendication 9, dans lequel l'applicateur comprend un dispositif de déclenchement.
